# EUROPEAN PATENT APPLICATION

(11) **EP 2 085 480 A1**
(43) Date of publication of application: **05.08.2009**
(21) Application number: 07823022.4
(22) Date of filing: 29.10.2007
(51) Int. Cl.: C12N 15/82, C12N 5/10, A01H 5/00

(54) **USE OF THE OCP3 MUTATION TO REGULATE DROUGHT RESISTANCE IN PLANTS**

(30) Priority: 31.10.2006 ES 200700128
(71) Applicant: Calantia Biotech, S.l., 46010 Valencia (ES)
(72) Inventor: RAMIREZ GARCIA, Vicente, E-46022 Valencia (ES); COEGO GONZÁLEZ, Alberto, E-46022 Valencia (ES); VERA VERA, Pablo, E-46022 Valencia (ES)
(74) Representative: Gonzalez-Alberto Rodriguez, Natalia
(86) International application number: PCT/ES2007/000616
(87) International publication number: WO 2008/053059

(57) **Abstract**

The invention relates to the technical field of plant biotechnology and, more specifically, to the use of the OCP3 gene as a regulator of drought resistance in plants and to the resulting plants having said drought resistance or increased drought tolerance.

## Description

The present invention is comprised within the technical field of plant biotechnology and it specifically relates to the use of the *OCP3* gene as a regulator of drought resistance in plants and to the plants obtained with said drought resistance or increased drought tolerance.

### State of the Art

Drought is probably the most important problem of modern agriculture, being the cause of substantial losses year after year. Prolonged exposure to a water shortage causes devastating and irreversible damage in the plant which often results in a reduction in the yield of crops and occasionally in their death.

During drought there is an increase in solute concentration due to a loss of water which in turn makes the water potential of the plant drop. This causes, in its initial stages, a destabilization of the membrane system (including the plasma membrane) as a whole and in turn the disruption of physiological and biochemical processes which are very important for cell homeostasis, among which photosynthesis should be emphasized due to its special relevance in plant organisms (*Holmberg and Bülow, 1998*). In fact, in drought conditions or in limited watering situations the photosynthetic rate can drop to levels that are so low that they can compromise the synthesis of the sufficient amount of ATP necessary to keep cell metabolism balanced and this can entail the death of the cell. In addition to the drop in the photosynthetic rate during the drought process, light continues to impinge on and excite chloroplasts. This therefore leads to an increase in the synthesis and accumulation of reactive oxygen species which inevitably participate in the generation of the aforementioned cell damage and thus lead to accelerated cell deterioration (*Holmberg and Bülow, 1998*).

Plants have developed sophisticated defense and adaptation strategies to deal with all these complex physiological and biochemical processes which are induced or are associated to drought. One of the earliest strategies is that of preventing an excessive loss of water through the activation of signaling cascades yielding an increase in abscisic acid (ABA) levels and the corresponding closure of the stomata; all of this being mediated by an increase in cytosolic Ca²⁺ levels in guard cells (*McAinsh et al., 1990*). In addition to this cell mechanism, and in the event that the drought period is prolonged over time, the plant furthermore starts up other mechanisms of adaptation and protection for the purpose of keeping its metabolism within sustainable limits. This involves the activation of different signaling pathways resulting in the expression of a cluster of genes encoding proteins which could work as antioxidants and osmoprotectants for the purpose of protecting and/or repairing cell damage caused by the decrease in the water potential (*Courtois et al., 2000*).

ABA is the main hormone regulating all these adaptation and protection processes orchestrated in response to water stress. It is essentially in charge of keeping a water balance in the guard cells of stomata and increasing osmotic stress tolerance through the regulation of a large number of genes. Thus, mutants deficient in ABA synthesis or perception show a drastic decrease of water stress tolerance, being incapable of effectively responding to relatively short water shortage periods (*Xiong* et *al., 2001*). In addition, most of these ABA-regulated genes have in their promoter regions a common regulatory sequence called ABRE (ABA-responsive element) element or box. Proteins which show capacity to bind to these ABRE sequences have been identified and said proteins are called AREB (ABA-responsive element binding) or ABF (Auxin binding factors) factors. These are bZIP (Basic leucine zipper) type transcription factors and work as transcriptional activators in response to ABA (*Uno et al., 2000; Choi et al., 2000*). The overexpressions of some of these elements, as occurs with the ABF3 factor or the ABF4 factor, confer to transgenic plants an increase in drought tolerance which is accompanied by an alteration in the expression of stress-responsive genes, such as the *rd29B, rd22, rab18, ABI1* and *ABI2* genes (*Kang et al., 2002*). In addition, the MYB and MYC proteins are very important transcriptional regulators which also participate as activators in ABA-dependent regulation systems (*Abe et al., 2003*).

In addition to this transcriptional regulation cascade, the so-called reactive oxygen species or ROS have a predominant role in the mechanisms of perception and adaptation to drought (*Sanders et al., 1999*). Thus, ROS are induced by ABA and it is proposed that they could act as intermediates of the signaling mediated by this hormone in response to drought (*Pei et al., 2000*).

There is, however, another group of genes as is the case of the *rd29A* (responsive to dehydration) gene, the expression of which is induced in response to drought although in an ABA-independent manner (*Seki et al., 2003*). These genes usually have in their promoter region a conserved CIS sequence or element to which regulatory proteins called DRE (Dehydration-responsive element) or CRT (C-repeat) bind (*Yamaguchi-Shinozaki and Shinozaki, 1994, Baker et al., 1994*). These proteins are transcription factors belonging to the ERF/AP2 (Ethylene-responsive element binding factor/Apetala2) family (*Weigel, 1995, Seki et al., 2003*). There are two groups of DRE/CRT proteins, the DREB2 group, involved in the response to drought, and the DREB1 group, furthermore involved in the response to cold (*Liu et al., 2000*). It has been demonstrated that by overexpressing both DREB1A and a modified version of DREB2A, a large increase is achieved in the drought tolerance of transgenic plants which can be attributed to an increase in the induction of the expression of stress-responsive genes, such as the aforementioned *rd29A* gene (*Liu et al., 2000; Gilmour et al., 2000; Sakuma et al., 2006*). However, these plants have a pleiotropic phenotype in which even though they are resistant to the imposed stress, they experience serious disorders in their development pattern; they are dwarf plants with aberrant leaf morphology. Subsequent works have shown an alternative technology for palliating this pleiotropic aspect of said overexpression by means of using an inducible system for overexpressing this type of gene, obtaining more drought-resistant plants without reducing their growth (*Kasuga et al., 1999; Sakuma et al., 2006).*

### Description of the Invention

A first object of the present invention relates to the functional involvement of the *OCP3* gene, encoding a Homeobox type transcription factor, as a regulatory node of the response of plants to drought, i.e., the use of the *OCP3* gene as a regulator of drought resistance in plants. In the present invention it is demonstrated that with the generation of plants having the capacity to synthesize functional OCP3 proteins by means of chemical mutagenesis with EMS blocked, plants are obtained with a large increase in the resistance to prolonged drought periods, as is evident in the *ocp3* mutant. It is also demonstrated that the inhibition of the expression of said gene in plants by means of gene silencing approaches, in the same way as the loss of function associated to the *ocp3* mutant, also generates resistance to prolonged drought periods.

Therefore, a second aspect of the present invention would consist of the plants obtained with decreased expression of the *OCP3* gene, having a large increase of the resistance to prolonged drought periods.

In the beginning, the *ocp3* mutant was isolated from a population mutagenized with ethyl methanesulfonate (EMS) and coming, in turn, from a transgenic *Arabidopsis thaliana* line - designated as line 5.2- in which the promoter of the *Ep5C* gene of *Lycopersicon esculentum* (initially described in *Coego et al., 2005a)* directed the expression of the *uidA* (GUS) gene, encoding the β-glucuronidase enzyme (*Jefferson et al., 1987*). In this screening, several *ocp* (overexpressor of cationic peroxidase) mutants were isolated. Specifically, the recessive *ocp3* mutant has a constitutive expression of the *GUS* gene, giving an intense and homogeneous blue color in the entire plant when a GUS staining is performed with the X-Gluc substrate. One of the most important characteristics of this mutant is its indisputable resistance to necrotrophic type pathogens (*Coego et al., 2005b*).

### ocp3 plants have a high drought resistance.

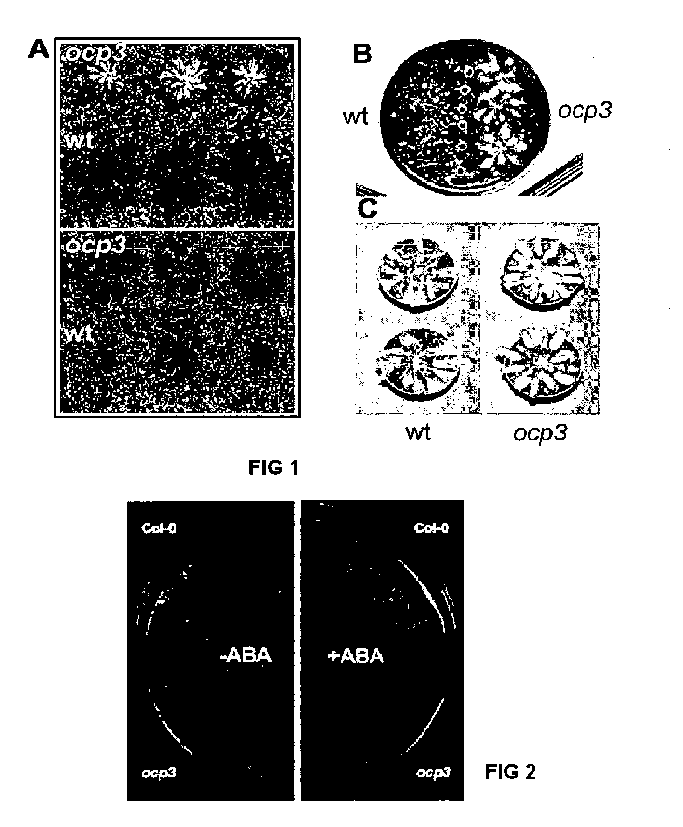

A thorough molecular characterization of the *ocp3* mutant revealed some characteristics of agronomic interest inherent to the mechanism of loss of function which can be attributed to the *ocp3* mutation. Within these characteristics, the observation of a high resistance to prolonged exposures to absence of water or watering when compared with non-mutant plants with a wild type genotype (Col-0), hereinafter wt (Wild type) plants, is emphasized. Thus, if wt plants of between 4 to 6 weeks of age grown in normal short day conditions are not watered for 12 days, they irreversibly deteriorate; in contrast this does not occur in the *ocp3* mutant. Figure 1 shows representative plants of each genotype: wt and *ocp3* grown either individually (in independent pots) or grouped in the same pot. As can be observed, the wt plants have pronounced wilting, even ending up dying, whereas the *ocp3* mutant plants remain apparently healthy or unchanged after the prolonged drought period. Even when replacing the watering regime after said prolonged drought period, the wt plants did not recover, whereas the *ocp3* plants continued their normal development (Figure 1). These results demonstrate that the loss of function of the *OCP3* gene, through the recessive *ocp3* mutation, is sufficient to confer to the *Arabidopsis thaliana* plants an evident resistance to drought and argues in favor of the OCP3 protein being able to work as a negative regulator of an existing mechanism of plant adaptation for survival in prolonged water shortage or drought periods.

### The ocp3 mutant is hypersensitive to ABA.

There is a large amount of evidence of the involvement of ABA in the response of plants to water stress (*Zeevaart and Creelman, 1988; Davies and Jones, 1991; Koornneef* et *al., 1998; Hetherington, 2001; Schroeder et al., 2001*). Thus, for example, mutant plants insensitive to exogenous ABA application, as is the case of the *abi1* or *abi2* mutants, are incapable of effectively responding to a water absence regime, and said mutant plants wilt and die in relatively short time periods. Among the several effects triggered by the exogenous ABA hormone application to *Arabidopsis* plants, one of them is that of triggering a decrease in the root elongation capacity, an aspect which is furthermore reflected in a smaller size of the aerial part of the plants. Thus, the mutants insensitive to ABA, such as *abi1* and *abi2*, show a lower inhibition of the root development compared to that experienced by the Col-0 and Ler controls (*Koornneef et al., 1984*).

To study any possible effect of exogenous ABA application in the development of *ocp3* plants, seeds of said mutant were seeded in MS-Agar plates, as well as in MS-Agar plates supplemented with an ABA concentration of 0.8 µM, and always using the wt wild type genotype (Col-0) as control plants in these experiments. As shown in Figure 2, the development of the *ocp3* mutant is arrested in the first stages of germination if the latter occurs in the presence of ABA and therefore it has a greater sensitivity to the ABA hormone compared with the wt control (Col-0). Based on this it could therefore be deduced that the loss of function which can be attributed to the *ocp3* mutation yields a hypersusceptibility to the ABA hormone and therefore the normal function of the *OCP3* gene would be compatible with that of regulating any repression aspect of the ABA hormone transduction pathway, either in the sense of its synthesis or in that of its correct perception.

### The ocp3 mutant does not have alterations in the expression levels of dehydration-responsive genes.

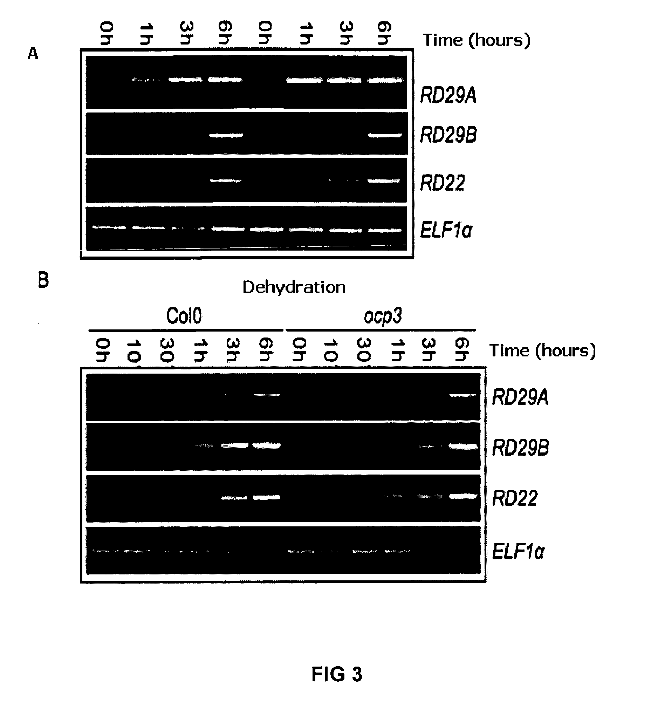

A possibility that would explain the greater sensitivity to ABA of the *ocp3* mutant as well as the increase in drought resistance could be that the mutation in said gene had caused a constitutive activation of the ABA-dependent signaling pathway and the final result of which would be the constitutive expression of dehydration-responsive genes which increase the protection levels against the damage caused by water stress and therefore this would aid the plants in better resisting this type of stress. For the purpose of studying this possibility, a molecular analysis was conducted, determining the expression levels (measured as accumulation of the corresponding mRNA by RT-PCR) of several signaling marker genes in response to dehydration, both of those genes activated by the ABA-dependent route, and of those genes the activation of which is independent of the ABA route. The *rd29B* and *rd22* genes were used as markers of the ABA-dependent pathway, whereas *rd29A* was used for the ABA-independent pathway. The levels of the transcripts of these genes at different times (0, 1h, 3h and 6h) were analyzed after incubating the plants in a liquid medium with 150 µM ABA. As shown in Figure 3A, there are no observable differences in the induction and accumulation levels of the transcripts corresponding to the markers used when the RNA populations obtained from wt (Col-0) plants and similarly treated *ocp3* plants are compared. This indicates that there is no observable defect in the signaling pathway mediating the induction of the expression of these genes by ABA and eliminates the consideration that the *ocp3* mutation is affecting said gene activation route.

To complement the molecular characterization, the levels of the transcripts of these same marker genes in response to dehydration were compared. The experiments were conducted as described in Sakuma *et al.* Col-0 and *ocp3* seedlings were grown *in vitro* in MS-Agar plates and after 3 weeks the seedlings were transferred to sterile Petri dishes without culture medium to thus cause their dehydration. The accumulation levels of the transcripts corresponding to the previously indicated marker genes were analyzed after 0, 10', 30', 1h, 3h and 6h of dehydration. The results (Figure 3B) demonstrate that *rd29B* and *rd22* as well as *rd29A* show a pronounced accumulation in dehydration inducing conditions but, at the same time, no differences are observed between the induction and accumulation levels of the corresponding transcripts when RNA populations obtained from wt (Col-0) plants and similarly treated *ocp3* plants are compared. All of this therefore indicates that the *ocp3* mutant does not show any defect in relation to the activation of the signaling cascades induced by these marker genes in response to dehydration either.

In summary, the results indicate that despite the fact that the *ocp3* plants show a marked sensitivity to ABA compared with the Col-0 plants in terms of inhibition of development, there are no differences in the inducibility of the expression of marker genes that are ABA-dependent (such as *rd29B* and *rd22*) or ABA-independent (such as *rd29A*). Therefore, the drought resistance shown by the *ocp3* mutant seems to be independent of the expression of these genes.

### The expression of the OCP3 gene is repressed in response both to exogenous ABA application and to dehydration.

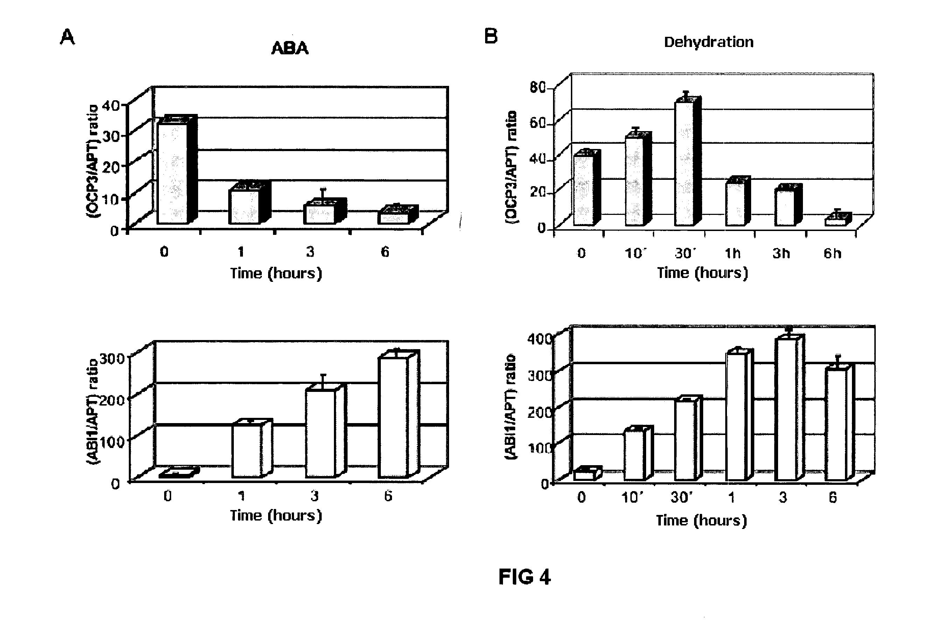

In both drought and dehydration conditions, plants respond by increasing the endogenous levels of ABA, which involves the activation of a signaling cascade having as a result a series of changes at the transcriptomic level. To study the possible effect that ABA may have in transcriptional *OCP3* regulation in wt plants, the relative levels of the mRNA corresponding to the *OCP3* gene is measured by quantitative RT-PCR (q-RT-PCR) at different times after applying ABA, as well as after subjecting the plants to dehydration stress. The *ABI1* gene was used as a control in the experiments, the expression of which is known to be induced in response to both ABA and dehydration. As can be observed in Figure 4, both as a result of applying 150 µM ABA and after imposing a water stress to the seedlings, a sharp drop of the accumulation levels of the transcript corresponding to the *OCP3* gene is observed. Said repression in the expression of the *OCP3* gene as a result of the treatment with ABA or of dehydration, already becomes evident at very short times. Furthermore, said drop is inversely proportional to the activation observed for the *ABI1* gene (Figure 4). This therefore indicates that the *OCP3* gene could be involved in an early signaling in response to the increase in the levels of ABA, such that its repression is necessary, although not necessarily sufficient to promote the activation of the transcriptional program demonstrated for the previously indicated reference genes, including the *ABI1* gene itself. In addition, the induction of the ABI1 protein, which works as a negative regulator of the response to ABA, would serve to modulate this intense transcriptional demand activated by the inductive conditions used.

### The ocp3 mutant does not show alterations in response to desiccation.

One of the first characteristic events occurring in plants after the perception of an increase in the levels of endogenous ABA as a result of prolonged drought periods is the closure of stomata, since ABA promotes the closure thereof and also inhibits the opening of closed stomata (*Schroeder et al., 2001*). Therefore, an indirect measurement of the capacity of plants to close stomata consists of determining the loss of fresh weight over time in leaves detached from the plant and placed under controlled environmental temperature and moisture conditions. Thus, mutants altered in the synthesis, perception and/or response to ABA are incapable of effectively closing stomata and, therefore, in said mutants there is a quicker decrease in the fresh weight in response to this desiccation process (*Allen* et *al., 1999; Finkelstein and Somerville, 1990; Koornneef et al., 1984; Leung et al., 1997*).

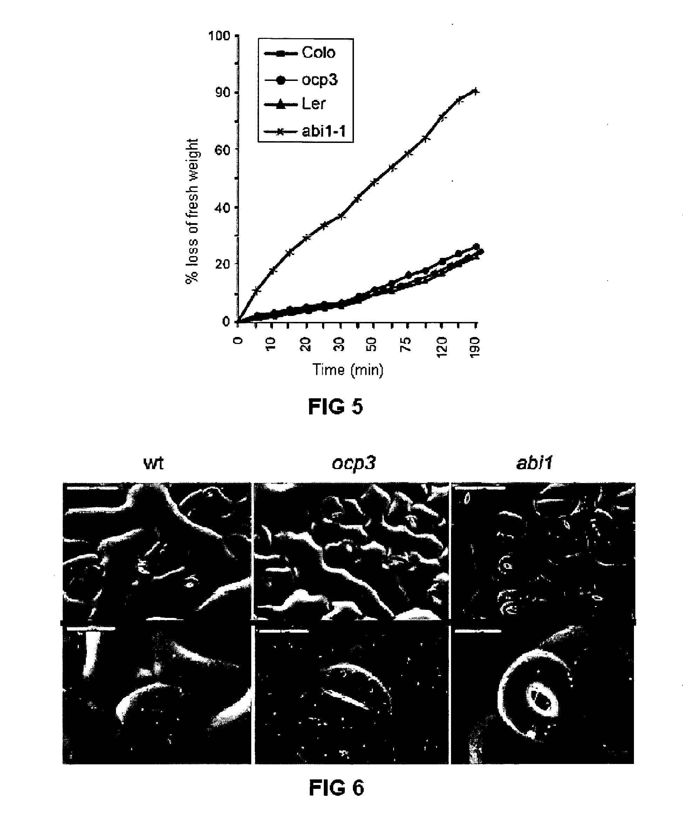

For the purpose of observing the behavior of *ocp3* plants in relation to this physiological aspect, this type of assay for measuring the dehydration rate is conducted and the data are in reference to those obtained with *abi-1* plants and the corresponding control (wt) plants; Col-0 as the parent of the *ocp3* mutant and Ler as the parent of the *abil-1* mutant. To that end, rosette leaves of plants of each of the genotypes were cut, divided into three replicas and placed in sterile Petri dishes in a laminar flow cabinet to cause their desiccation, measuring the loss of fresh weight at different times. As can be observed in Figure 5, the *abi1-1* mutant has an abrupt loss of fresh weight with losses close to 80% of its fresh weight three hours after the desiccation was started. In contrast, the Ler and Col-0 controls have only lost 20% of their original fresh weight, as occurs with the samples from the *ocp3* mutant. Thus, despite the pronounced differences observed in response to drought between the *ocp3* mutant and the wt plants, there are no significant differences in response to desiccation between said plants. This difference in the behavior, high drought resistance in plants grown in pots but normal behavior to quick desiccation by the *ocp3* plants, could be due to the fact that the two types of stress assayed (drought and desiccation) have a different signaling mechanism and precisely the *ocp3* mutant reveals said difference.

### The ocp3 mutant does not have alterations in the arrangement and number of stomata

According to the results obtained, and given that the number of stomata, their arrangement, and also the degree of opening/closure thereof can have an essential role in the response of the plants to water stress, the possibility of *ocp3* showing any stomatal alteration was considered. After a scanning microscopy analysis (Figure 6), no differences were observed between *ocp3* plants and wt plants or in the arrangement or amount of stomata throughout the leaf lamina. Significant differences were not found either in the degree of opening of the stomata between the *ocp3* plants and the wt plants when they are grown in normal temperature, lighting and watering conditions (Figure 6), although both of them were drastically different from that observed for the *abi1* mutant, which showed permanent stomatal opening and which explains the behavior observed for the *abi1* plants against dehydration.

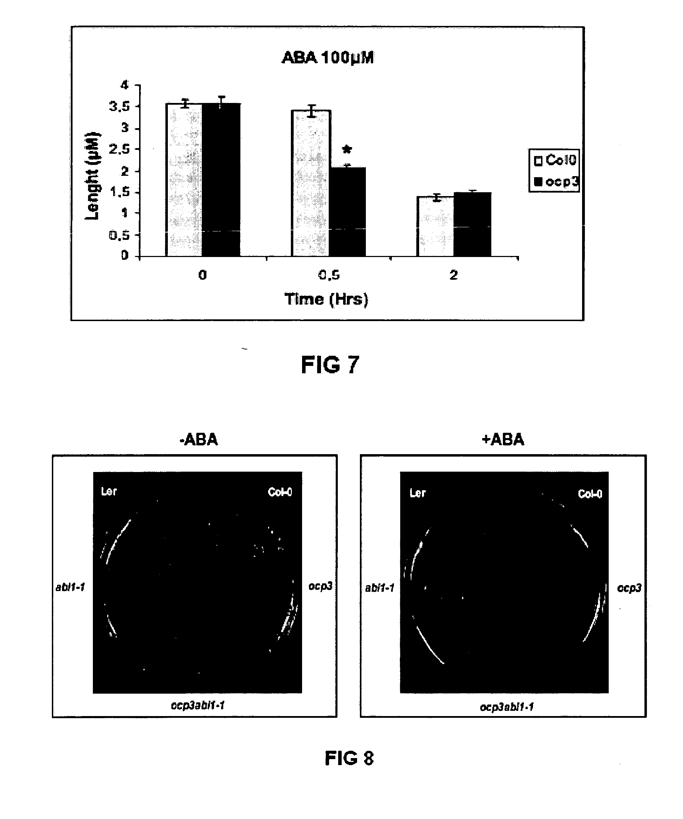

There are different stimuli modifying the rate as well as the degree of opening/closure of the stomata and, among them, subjecting the plants to drought or depriving them of light should be emphasized, and both signalings are always directly related to an increase in the endogenous levels of ABA, a hormone determining the closure of stomata under inductive or stress conditions. To determine if there is any defect in ABA-mediated signaling in the *ocp3* mutant, the decision was made to compare the stomatal closure rate between *ocp3* plants and Col-0 plants, occurring after direct ABA application in epidermal samples (peelings) obtained from rosette leaves. As can be seen in Figure 7, the presence of ABA causes an accelerated closure of the stomata in the *ocp3* plants which was already significantly observable 30 minutes after applying ABA and in situations in which the wt control has still not started the process for closing its stomata. However, these differences disappear 2 hours after applying ABA, at which time the stomata of the *ocp3* plants and the wt plants are completely closed. Therefore, it is inferred from these experiments that the *ocp3* mutant is more sensitive to the ABA hormone and this translates into an early response to this hormone which is manifested with an accelerated stomatal closure. The greater susceptibility to ABA is consistent with the hypersusceptibility shown by the seedlings when they are germinated and grown in the presence of ABA (Figure 2). Furthermore, said hypersusceptibility to ABA could explain the drought resistance phenotype, since drought causes increases in ABA synthesis which in turn lead to the closure of stomata to prevent the loss of water by evapotranspiration. Thus, since the *ocp3* mutant is more sensitive to ABA, it would accelerate the closure of its stomata with a drought signal and this promptness in the response could provide it with an advantage to resist the metabolic drawbacks derived from water privation better or for more time.

### The abi1-1 mutation eliminates the resistance of ocp3 plants to drought.

The involvement of the ABI1 phosphatase 2C in the ABA-dependent signaling pathways is clear and has been well studied (*Koornneef et al., 1984; Leung et al., 1997; Allen et al., 1999; Merlot et al., 2001; Mishra et al., 2006*). In fact, most of the phenotypes dependent on this hormone are reflected in the dominant *abi1-1* mutant. These mutant plants are hypersusceptible to drought and to dehydration since they are incapable of closing stomata due to having the ABA-mediated signaling constitutively repressed. Furthermore, *abi1-1* plants are insensitive to ABA and therefore do not show induction in the expression of the marker genes neither in dehydration conditions nor in response to exogenous ABA application.

For the purpose of determining if the drought resistance conferred by the *ocp3* mutation is ABI1-dependent, the *ocp3* mutation was introgressed into a mutant *abi1-1* background, thus generating the *ocp3 abi1-1* double mutant and the response of these plants in different situations was analyzed.

On one hand, the growth of the *ocp3 abi1-1* double mutant in MS plates supplemented with 0.8 µM ABA was studied and the effect that it exerted on the growth of seedlings was compared to the Col-0, *ocp3* and *abi1-1* genotypes. As can be observed in Figure 8, the *ocp3 abi1-1* double mutant behaves like *abi1-1,* and therefore shows suppression of the hypersusceptibility to ABA which can be attributed to the *ocp3* mutation.

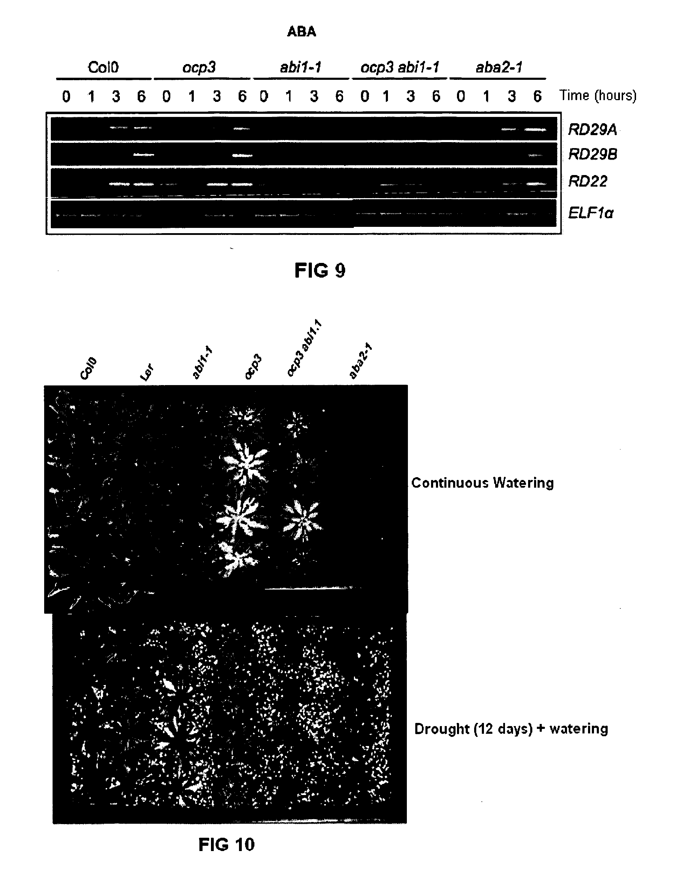

On the other hand, by analyzing the levels of the mRNA of the previously used ABA pathway marker genes, it was observed that the plants carrying the *ocp3 abi1-1* double mutation have a reduced induction of the expression of said marker genes in contrast to that observed with the wt or *ocp3* plants. The *ocp3 abi1-1* double mutant behaves like the *abi1-1* mutant, which also has a reduced activation of the expression of the reference genes in response to ABA (Figure 9). As can be seen both in the case of *abi1-1* and of the *ocp3 abi1-1* double mutant, the induction of the expression of the *rd29A* gene is greatly reduced, whereas in the case of the *rd29B* and *rd22* genes, it is virtually annulled. These results are consistent with those obtained in previous works in which the induction of the expression of marker genes that are both ABA-dependent, such as *rd29B,* and ABA-independent, such as *rd29A,* is decreased in the *abi1-1* mutant (*Nordin et al., 1993; Shinozaki et al., 2000; Umezawa et al., 2006*). In these experiments, the *aba2-1* mutant (Figure 9) was used as an internal control to verify that the ABA application and dose used have been effective. The *aba2*-*1* mutant does not synthesize ABA (*Léon-Kloosterziel et al., 1996*) and therefore any induction of expression of the previously used ABA-dependent genes can only be attributed to the exogenous ABA applied and not to any other internal disturbance which may result from the different genetic backgrounds used.

Finally, the behavior of the Col-0, *ocp3, abi1-1* and *ocp3 abi1-1* plants in response to drought was analyzed. Figure 10 shows a group of representative plants of each genotype which have been watered for two days, after having remained deprived of watering for 12 days (Figure 10) and the comparison with representative plants of each genotype which have not been subjected to said water stress and which have been watered normally (Figure 10). As can be seen in this Figure, the wt plants (in their respective Col-0 and Ler backgrounds) show wilting and stress symptoms, characteristic of watering privation and preceding the complete collapse thereof if the stress period is prolonged over time. Likewise, the *abi1-1* mutant and the *aba2-1* mutant, which have an altered ABA perception and synthesis, respectively, show an increased susceptibility to the imposed water stress -with an earlier and more pronounced manifestation of the characteristic symptomatology - and denotes the importance of this hormone in this type of response. In contrast, as expected, the *ocp3* mutant shows an evident behavior of resistance to said stress. However, the *ocp3 abi1-1* double mutant shows a behavior similar to that of the *abi1-1* single mutant with pronounced wilting which is again greater than that observed in the wt (Col-0) plants and from which they do not seem to recover after replacing the watering (Figure 10). The loss of the drought resistance demonstrated by the *ocp3 abi1-1* plants indicates, therefore, that for the manifestation of the resistance phenotype which can be attributed to *ocp3* it is absolutely essential to maintain the ABA pathway intact through the ABI1 regulator. From the foregoing it can furthermore be derived that in wild type conditions the ABI1 and the OCP3 protein can be interacting to mediate a behavior such as that observed in wt plants for the perception and response to drought.

### OCP3 interacts physically with ABI1.

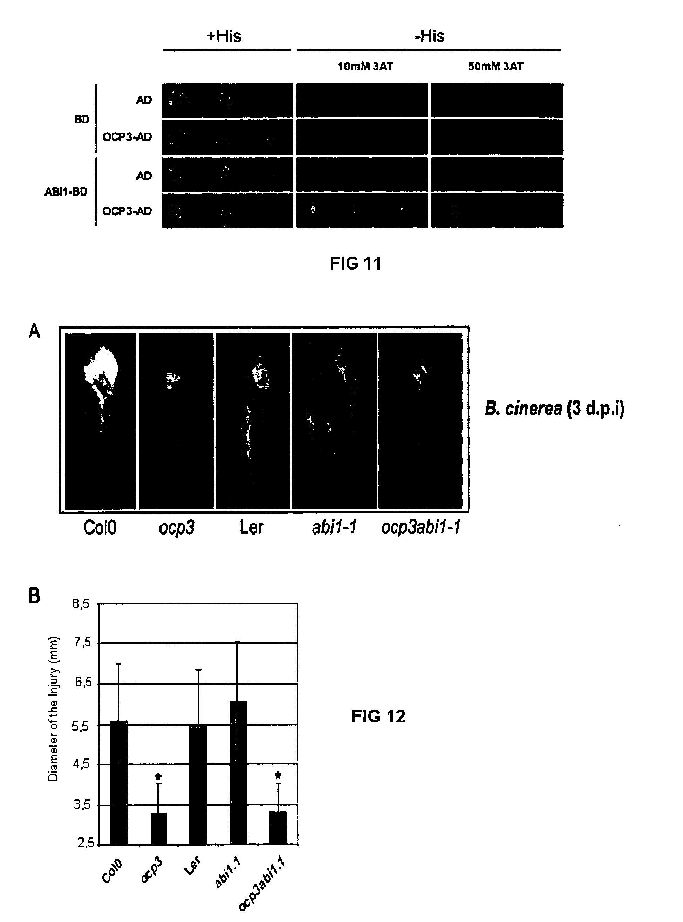

The identification of proteins interacting with OCP3 can be highly relevant for knowing the phenomena triggered in plants against different types of stress such as drought and the response to necrotrophic fungi in addition to being able to establish a possible link between them. To determine the existence of these interactions, the yeast "two-hybrid" technique was used (*Ausubel, 1987*). With this technique, the expression of a gene allowing the survival of yeasts in a culture medium deficient in a nutrient is under the control of a regulatory region or promoter of a gene which is only activated with the formation of a stable protein complex between the two proteins under study. In this case, the OCP3 protein was fused to the *GAL4* gene-activating domain, and this fusion (OCP3-AD) was used as a primer for a gene library of proteins of *A.thaliana* fused to the *GAL4* promoter-binding domain (X-BD). These fusions were expressed in a yeast which had a vector in which the *GAL4* promoter directing the expression of the *HIS3* marker gene was located, such that the growth of this yeast in a histidine-free medium would only be restored when there is interaction between OCP3 and an X protein. Furthermore, a competitive HIS3 inhibitor such as 3-AT (3-aminotriazole acid) was used to increase specificity. One of the clones detected in the screening corresponded to the cDNA of the *ABI1* phosphatase 2C, which demonstrated the existence of an interaction between OCP3 and ABI1. In Figure 11 it can be observed how the joint expression of the ABI1-BD and OCP3-AD fusion proteins is capable of restoring the growth of the yeast, whereas either of the two separately, or the empty vectors, do not do this. Therefore, it can be concluded that ABI1 interacts with OCP3. This result is consistent with those described up until now, in which the gain of the genetic function of the ABI1 phosphatase 2C present in the *abi1-1* mutant is capable of suppressing the drought resistance of *ocp3* plants. *OCP3* encodes a Homeobox type transcription factor, and there are several cases described in the literature in which the functional modulation of a transcription factor by means of phosphorylation/dephosphorylation has been demonstrated (*Kaffman et al., 1994; Pines 1999; Whitmarsh et al., 2000; Nowak and Corces, 2000*). Therefore, and given that ABI1 is a phosphatase, this interaction would take place for the purpose of dephosphorylating OCP3, very possibly to thus modulate its function.

### The resistance to Botrytis cinerea conferred by the ocp3 mutation is ABI1-independent.

Previous works have described the resistance phenotype of the *ocp3* mutant against infections by necrotrophic-type pathogens (P200501035; *Coego et al., 2005b*). The *ocp3 abi1-1* double mutant generated in this work seemed to be a highly valuable genetic material to investigate the existence of any regulatory role of the ABA hormone in establishing a defensive response of resistance to this type of pathogen as shown by the *ocp3* mutant. This double mutant integrates, on one hand, the incapacity for the perception and therefore the non-transduction of ABA-mediated signaling through the *abi1-1* mutation, and on the other hand, a molecular alteration which can be attributed to the loss of function in the *ocp3* locus and which has, as an outstanding effect, a greater resistance to fungi such as *Botrytis cinerea* or *Plectosphaerella cucumerina*, in addition to the previously shown greater drought resistance.

For the purpose of analyzing a possible involvement of ABA in the resistance to necrotrophic pathogens, experiments of inoculation with *B.cinerea* of Col-0, Ler, *ocp3, abi1-1* and *ocp3 abi1-1* plants were conducted. Figure 12 shows the response of these plants 72 hours after the inoculation with a suspension of spores of the fungus and in which the normal susceptibility observed in the wt plants is comparable to what is observed in the *abi1-1* plants. This suggests, therefore, that the normal susceptibility of the wt plants to said necrotrophic fungus does not seem to depend on correct ABA hormone perception. Likewise, the increase in the demonstrated resistance characteristic of the *ocp3* mutant is not affected either when said mutation is analyzed in the presence of the *abi1-1* mutation in the *ocp3 abi1-1* double mutant.

In summary, these results show that the resistance to necrotrophic fungi inherent to the *ocp3* mutant follows a course independent from that of a correct ABA hormone perception and transduction. On the contrary, said independence contrasts with the absolute requirement for ABA shown by the *ocp3* mutant to manifest the drought resistance demonstrated in previous pages. Therefore, these results indicate a possible role of the *OCP3* gene as a regulatory node of defensive responses of plants against different types of stress such as drought and necrotrophic fungi, and which substance requires the involvement of the ABA hormone through the *ABI1* gene as a regulatory element of the same substance for drought resistance, but not for resistance to fungi.

### The silencing of the OCP3 homologous tomato gene (LeOCP3) reproduces the drought resistance phenotype in tomato plants.

Virus induced gene silencing or VIGS is a method used to transiently interrupt the function of a gene through the interference of its mRNA (*Baulcombe, 2004*). Although the exact mechanism is not well known, this tool uses the natural capacity of plants to suppress the accumulation of foreign RNA as a mechanism of defense against viral infections (*Dawson, 1996*). The generation of recombinant viral vectors carrying specific sequences of endogenous plant genes has allowed successfully suppressing the expression of different genes, becoming a powerful tool for studying gene function (*Burton* et *al., 2002*), since the phenotype shown by the plants silenced by VIGS is comparable to that of a loss of function. A number of effective viral vectors for studying gene function in different plant species such as *Nicotiana benthamiana* (*Liu* et *al., 2002b), Lycopersicon esculentum* (*Ekengren et al., 2003; Liu et al., 2002a), Petunia* (*Chen et al., 2004*) or *Solanum tuberosum* (*Brigneti et al., 2004*) have been developed .

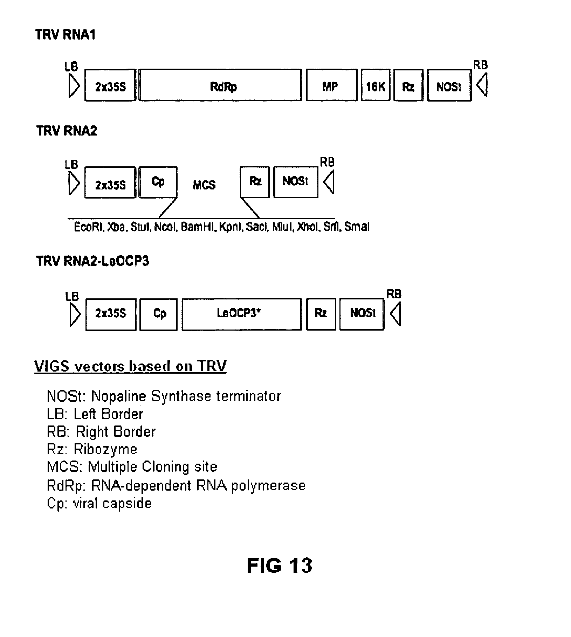

VIGS technology was used for the purpose of studying if the effect of the suppression of *OCP3* also increases the resistance of the plants in other plant species with a greater agronomic interest. Particularly, the second-generation VIGS vector based on the TRV virus (Tobacco Rattle Virus), a bipartite RNA virus, was used (*Liu et al., 2002a*). This vector has been designed such that there is a multiple cloning site in TRV RNA2 in which a specific sequence of about 344 bp of the *OCP3* homologous tomato gene (*LeOCP3*) (Figure 13) was inserted.

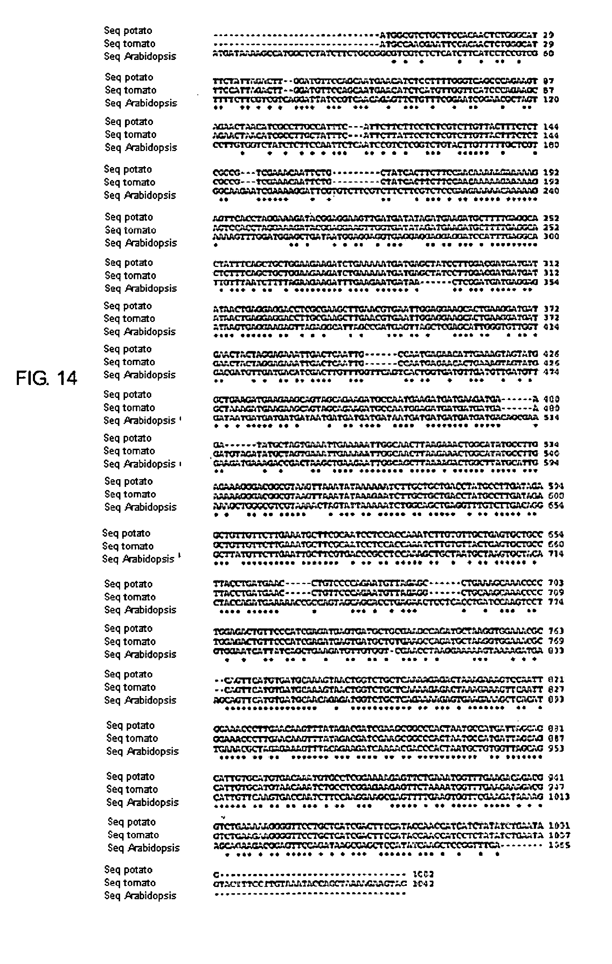

The orthologous tomato gene (*Lycopersicon esculentum*), hereinafter *LeOCP3*, was cloned from a potato (*Solanum tuberosum*) gene sequence which appeared in the Genebank database (accession number BQ112211) and showed an amino acid sequence identity of 48.3% with OCP3 of *Arabidopsis thaliana.* Based on the potato *OCP3* nucleotide sequence, specific oligonucleotides (oligonucleotides PoRT1 with SEC. ID. No. 1 and PoRT2 with SEC. ID. No. 2) were designed and used as oligonucleotides in a PCR reaction on a cDNA sample obtained from tomato leaf RNA. The product of said PCR reaction was cloned into vector pTZ57 and said cloned DNA product which would be referred to as *LeOCP3* (Figure 14) was sequenced. The sequence resulting from said amplification was 95% identical to the potato sequence. A 330 bp fragment (*LeOCP3**) was amplified from the tomato clone by PCR with the specific primers of the tomOCP3EcoRI and tomOCP3XhoI tomato sequence (incorporating EcoRI and Soy cleavage sites, respectively) and after digesting with these enzymes, this fragment was ligated into vector TRV RNA2, thus generating construct TRV RNA2-LeOCP3* (Figures 13 and 14).

When leaf sectors or cotyledons of tomato plant leaves are co-infiltrated with *Agrobacterium tumefaciens* crops (agroinfiltration) containing the constructs TRV and TRV RNA2-LeOCP3*, respectively, the plant cells recognize the transcribed RNAs as foreign and silence them. This silencing is effective for both viral RNAs and for endogenous *LeOCP3* RNA, and furthermore the silencing mechanism becomes systemic, covering the entire plant which was initially agroinfiltrated locally.

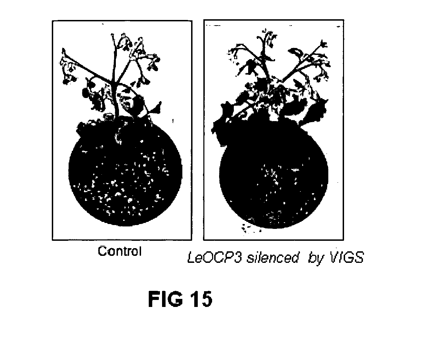

To evaluate the effect that the silencing of the Le*OCP3* gene may have on the drought resistance of tomato plants, Micro Tom variety tomato plants the cotyledons of which were co-infiltrated with *A.tumefaciens* crops containing, on one hand, TRV RNA1 and, on the other hand, TRV RNA2 with a 344 bp fragment specific for *LeOCP3,* were used. The agroinfiltrations were performed in batches of 20 seven day-old plants. Cotyledons of Micro Tom plants with empty TRV RNA1 and TRV RNA2, plants with TRV RNA1 or TRV RNA2 alone, and plants without any of these vectors were agroinfiltrated as internal controls for said experiments. To confirm that the silencing was effective, a variant of this viral vector including a fragment of about 500 bp of the *LePDS* gene (*Phytoene desaturase*) was furthermore used. This gene encodes a key enzyme in carotenoid biosynthesis, therefore a reduction in the expression thereof results in an easily detectable phenotype such as the loss of pigment of the leaves (*Liu et al., 2002a*). Twenty days after the agroinfiltration, the watering of the plants was eliminated and the drought resistance phenotype was gradually evaluated. The plants were photographed fifteen days after the watering privation. The non-agroinfiltrated control plants as well as those agroinfiltrated with the empty vectors (without the *LeOCP3* sequence) showed a characteristic drought phenotype which is manifested with wilting and pronounced leaf dehydration. In contrast, the plants agroinfiltrated with *A.tumefaciens* carrying TRV RNA1 + TRV RNA2-*LeOCP3* were capable of resisting this drought period without experiencing considerable damage (Figure 15). In addition, after 10 days and in all the cases analyzed, the silencing of the *LePDS* gene in the plants co-infiltrated with TRV RNA1 and TRV RNA2-PDS was evident, a pronounced chlorosis occurring in the leaves which indicated that the gene silencing based on the recognition of specific sequences was effective in all the cases.

In short, it seems clear that the silencing of the *LeOCP3* endogenous tomato gene is capable of phenocopying the *ocp3* mutant of *A.thaliana*, indicating that the regulatory function of the *OCP3* gene is conserved in two species that are so distant. Therefore, the decrease in the expression levels of the *OCP3* gene could be a general mechanism of plants to respond to drought by decreasing the damage experienced due to this stress. Obtaining plants with a decreased expression in this gene can provide a serious tool for obtaining crops with a greater drought tolerance.

Therefore, in the present invention it is demonstrated that the loss of function of the *OCP3* gene in *Arabidopsis thaliana*, as observed in the *ocp3* mutant (a mutant induced by the chemical agent ethyl methanesulfonate (EMS)), confers a marked resistance to prolonged drought periods. Said resistance occurs despite not having an altered induction of the expression of drought-inducible genes, and this is valid for both genes the induction of which is documented as ABA hormone-dependent and for other genes the induction of which is ABA hormone-independent, and which are normally used as molecular markers measuring the impact of water stress in plants. In this sense, it is also demonstrated that in wild type plants the expression of the *OCP3* gene is negatively and quickly controlled by the ABA hormone, as well as by the actual dehydration; and said negative regulation occurs following a time pattern that is similar, although reverse, to the induction of the *ABI1* gene, one of the main regulators of ABA-mediated signaling. In addition to the capacity to resist prolonged drought periods, when the normal function of the *OCP3* gene is mutated or lost, the present invention also demonstrates that for this to occur it is necessary to maintain correct ABA hormone perception intact through the correct functioning of the ABI1 protein.

The present invention also demonstrates the existence of a physical interaction between the ABI1 and OCP3 proteins, establishing a new role for OCP3 as a node modulating the response of the plants to drought.

Another aspect shown by the present invention is the independence from ABA in the resistance to necrotrophic fungi conferred by the absence or loss of function of the *OCP3* gene. The latter demonstrates that the capacity to confer drought resistance and resistance to fungi is through different pathways, but they both converge in the actual function of this gene the normal function of which is to act as a transcriptional repressor of both signal transduction pathways. Furthermore, the manipulation of the gene homologous to *OCP3* in other plants, as has been demonstrated for the case of tomato, by means of silencing the orthologous gene by VIGS technology, reproduces the drought resistance phenotype observed in *Arabidopsis thaliana* through the *ocp3* mutant. Therefore, the present invention demonstrates that the elimination or inhibition of the normal function of the gene or of the OCP3 protein is an instrument for controlling, conferring and inducing increases in the resistance of the plants to drought periods as well as for controlling, conferring and inducing long-lasting resistances to the attack by necrotrophic fungi.

### Description of the Drawings

Figure 1. Assay of drought resistance of wt and *ocp3* plants. (A) 6 week-old plants were not watered for 13 days and after this time the normal watering regime was restored (bottom panel). Appearance of the plants watered normally (top panel). (B) 4-week old plants, of the wt genotype (left) and *ocp3* genotype (right) were not watered for 12 days and after this time the normal watering regime was restored. (C) 5 week-old wt and *ocp3* plants grown individually; they were not watered for 14 days and after this time the normal watering regime was restored. Observe the unchanged phenotype after the different treatments of water privation in the case of the *ocp3* mutant and, in contrast, the deleterious aspect that said treatment has on the wild type wt plants.
Figure 2. Effect of ABA in the development of *ocp3* plants compared with wt (Col-0) plants. The seeds were sterilized, stratified at 4°C for 4 days and seeded in plates with MS-Agar medium and MS-Agar supplemented with 0.8 µM ABA. The photographs were taken 17 days after the seeding.
Figure 3. Analysis of the expression by RT-PCR of *rd29A, rd29B* and *rd22* water stress marker genes in Col-0 and *ocp3* plants, using the *ELF1*α gene as a control. The plants were grown in MS-Agar plates for 3 weeks and the RNAs were extracted from samples collected at different times. (A) Expression levels after exogenous 150 µM ABA application in a liquid culture. (B) Expression levels 10 minutes, 30 minutes and 1, 3 and 6 hours after subjecting the plants to dehydration.
Figure 4. Relative expression levels of *OCP3* and *ABI1* in response to exogenous 150 µM ABA application (A) and dehydration (B) measured by real-time PCR. The graphs show the levels of the *OCP3* and *ABI1* transcript, both of them referring to those of the *APT* gene and represented as the mean of three independent measurements. The error bars represent the standard deviation. The experiments were repeated at least three times with similar results.
Figure 5. Loss of fresh weight of leaves subjected to desiccation. Three replicas per genotype were used, causing their desiccation by cutting them and placing them in Petri dishes in a laminar flow cabinet. The loss of weight was determined according to the difference between the weight of the leaves at time 0 and each of the times indicated. The *abi1-1* mutant (-X-) dries very quickly, whereas there are no significant differences between Col-0 (-■-), Ler (-▲-) and *ocp3* (-●-)
Figure 6. Leaf surface analysis by scanning microscopy of leaves from wt, *ocp3* and *abi1* plants. The bottom panel is a magnification in which representative stomata for each selected genotype are shown and in which the stomatal opening corresponding to the leaf samples of *abi1* plants is evident. The bars represent 100 microns (top panel) or 10 microns (bottom panel). No differences are observed between the degree of opening/closure or in the number of stomata between the wt and *ocp3* samples.
Figure 7. Measurement of the stomatal closure induced by ABA (100 µM) in *ocp3* and wt plants. Rosette leaves were detached from 6 week-old plants of the ocp3 and Col-0 genotypes and incubated in a solution (10 mM Mes-KOH pH 6.15 and 30 mM KC1) for 2 hours to induce the opening of the stomata. Then, 100 µM ABA was added to induce the closure of the stomata. The opening of the ostiole was measured at the different times indicated in epidermal peelings of the leaves with the different genetic backgrounds. Thirty minutes after the ABA application a greater closure of the stomata in the *ocp3* mutant is evident and statistically significant. The bars represent the mean of the experiments with at least 90 measurements per experiment. The error bars represent the standard error of the mean (SEM). The asterisks indicate significant differences according to a Student's t-test (p<0.001)
Figure 8. Effect of ABA in the development of the *ocp3abi1-1* double mutant. The *ocp3* and *abi1-1* parents in addition to Col-0 and Ler were used as controls. The seeds were treated in the same way as described for Figure 2.
Figure 9. Comparison of the induction by ABA of the expression of *rd29A, rd29B* and *rd22* water stress marker genes assayed by RT-PCR in RNA samples from Col-0, *ocp3, aba2-1, abi1-1* and *ocp3abi1-1* plants, and using the accumulation of the mRNA corresponding to the *ELF1*α gene as a control. The procedure was the same as that described for Figure 3. The induction of the expression of the marker genes is clearly decreased both in the *abi1-1* mutant and in the *ocp3 abi1-1* double mutant if compared with the levels obtained for *ocp3* or wt.
Figure 10. Assay of drought resistance of *ocp3, abi1-1* plants and the *ocp3 abi1-1* double mutant. Col-0 and Ler as genetic backgrounds of the respective mutants were used as controls, and the *aba2-1* mutant was used as a drought hypersensitivity control. 6 week-old plants were not watered for 12 days (bottom panel). Appearance of the plants watered normally (top panel). The *ocp3 abi1-1* double mutant shows a susceptible behavior similar to that observed in the *abi1-1* single mutant which clearly contrasts with the resistance exhibited by the *ocp3* plants.
Figure 11. Interaction between OCP3 and ABI1. Growth of yeasts carrying the different plasmid combinations in selective medium with and without histidine and different concentrations of 3AT, an HIS3 inhibitor. Only the yeast producing the OCP3-AD and ABI1-BD fusion proteins is capable of growing in the absence of histidine and high concentrations of 3AT, indicating that both proteins interact.
Figure 12. Assay of resistance to *B. cinerea* of Col-0, *ocp3*, Ler, *abi1-1* and *ocp3abi1-1* plants. (A) Representative photographs of the injury developed by the fungus 72 hours after the infection in the different genotypes. (B) Measurement of the diameter of the necrosis caused by *B.cinerea* in the different genotypes. The mean of the measurements is represented in the graph. The error bars represent the standard deviation. The asterisks indicate significant differences with respect to the Col-0 control.
Figure 13. VIGS vectors used in the assays. Vector TRV RNA2-LePDS used as a silencing control in the assays is identical to TRV RNA2-LeOCP3 but substituting LeOCP3 with LePDS.
Figure 14. Alignment of the cDNA sequences of the *OCP3* gene of *A.thaliana* and the potato and tomato orthologs thereof made with the CLUSTAL W program (1.83). The asterisks indicate the nucleotides conserved in the 3 species.
Figure 15. Assay of drought resistance in Micro Tom tomatoes silenced for *LeOCP3* by means of *VIGS* technology. The photographs show a representation of the considerable resistance of the Micro Tom tomato plants agroinfiltrated with the vectors TRV RNA1 + TRV RNA2-*LeOCP3* (right) with respect to the controls agroinfiltrated with the empty vectors TRV RNA1 + TRV RNA2 (left).

### Detailed Description of an Embodiment

### Growth of the plants and plant material used.

The plants were grown in Jiffy-7 compacted substrate (Clause-Tezier Iberica, Valencia, Spain) at 23°C with a photoperiod of 10 hours of light and 14 of darkness. For the *in vitro* culture, the seeds were sterilized by adding 70% ethanol for 2 minutes and commercial 30% lye for 7 minutes. Subsequently, 5-7 washes with sterile water were performed and they were stratified at 4°C for 4 days. The culture medium used was Murashige & Skoog (MS) (Sigma) supplemented with sucrose (10g/L) and Agargel (Sigma) in the indicated cases. The plants were grown for the indicated time periods at 22°C, a photoperiod of 16 hours of light and 8 of darkness, with a light intensity of 150-200 µEm⁻² s⁻¹.

The mutants on a genetic background of the Col-0 ecotype of *A. thaliana* (L.) Heynh.: *ocp3* and *aba2-1,* and the *abi1-1* mutant on a genetic background of the Lansberg erecta (Ler) ecotype were used.

The tomato plants were grown as described in *Coego et al. (2005^{a}).*

### Gene constructs.

The construct used in the yeast two-hybrid screening was generated by cloning the cDNA of OCP3 into vector pAS2.1 (Clontech) amplified with the primers OCP3-1 with the sequence identified as SEC. ID. NO. 19; and OCP3-2 with the sequence identified as SEC. ID. NO. 20; generating EcoRI and SmaI cleavage sites respectively.

To clone the *LeOCP3* gene and the *LeOCP3** fragment, primers were designed which included cleavage sites for the SacI and NcoI restriction enzymes at the 5' and 3' ends, respectively:

| |
|---|
| PoRT1: SEC. ID. NO. 1 |
| PoRT2: SEC. ID. NO. 2 |
| tomOCP3EcoRI: SEC. ID. NO. 3 |
| tomOCP3XhoI: SEC. ID. NO. 4 |

Pwo SuperYield DNA Polymerase (Roche) was used for the PCR amplification reactions. The conditions used for the PCR reactions are detailed in the following table:

| Construct | Annealing T | Extension time | No. of cycles | Size |
|---|---|---|---|---|
| pAS2.1:OCP3 | 55 | 2' | 22 | 1065 |
| LeOCP3 | 57 | 2' | 30 | 1042 |
| LeOCP3* | 58 | 1' | 20 | 330 |

The PCR products were digested with the corresponding restriction enzymes and ligated into the indicated vectors using T4 DNA Ligase (New England Biolabs). The constructs were subsequently checked by means of sequencing.

### Assays of dehydration and exogenous ABA application.

The plants were grown for 2-3 weeks in MS-Agar medium supplemented with 10g/L sucrose. For the dehydration experiments, the plants were placed in Petri dishes. Plants collected directly from the MS-Agar plates were used as time 0. In the case of the exogenous ABA application experiments, the plants were transferred to 50 mL of liquid MS medium under stirring supplemented with 10 g/L sucrose and in the indicated cases with 150 µM ABA (Sigma). After the different times, 4-5 plants per genotype (approximately 150 mg) were taken, eliminating the remains of medium, and frozen in liquid nitrogen.

### Desiccation Assay

For these assays, 6 week-old plants grown in short day conditions were used. Five-six rosette leaves of 3 plants per genotype were cut and placed in Petri dishes. The dishes were placed in a laminar flow cabinet and sequential weighing operations were performed after 5, 10, 15, 20, 25, 30, 40, 50, 60, 75, 90, 120, 150 and 180 minutes. Three independent replicas of different plants were generated for each genotype. The mean of the weight loss percentages of the three replicas together with their standard deviations were represented, considering the initial weight as 100%. The assay was repeated three times with comparable results.

### Analysis of the gene expression by RT-PCR

The semi-quantitative RT-PCR technique was used to determine the relative amounts of some mRNAs of interest. For the RNA extractions the starting material was approximately 150 mg of tissue, using Trizol (Invitrogen) in accordance with the protocol of the company. The total RNA was resuspended in 50 µL of RNAse-free water. The RNA (5 µg) was reverse-transcribed using the Revertaid H Minus First Strand cDNA Synthesis Kit (Fermentas) with the Random Hexamer Oligonucleotide primers supplied in the kit. Based on the cDNA obtained, the different PCRs were conducted with specific primers for each gene in a PTC-100 Peltier Thermal Cycler. The RT-PCR products were separated in a 3% agarose gel in 1X TAE. The primers used for the case of samples derived from *Arabidopsis thaliana* are shown in the following table:

| Gene | Sequence | Number of cycles |
|---|---|---|
| forward rd29A | SEC.ID.NO.5 | 19 |
| reverse rd29A | SEC.ID.NO.6 | |
| forward rd29B | SEC.ID.NO.7 | 23 |
| reverse rd29B | SEC.ID.NO.8 | |
| forward rd22 | SEC.ID.NO.9 | 22 |
| reverse rd22 | SEC.ID.NO.10 | |
| forward ELF1α | SEC.ID.NO.11 | 18 |
| reverse ELF1α | SEC.ID.NO.12 | |

### Real-time PCR

The samples were analyzed in triplicate and the real-time PCR reactions were performed using Sybr Green PCR Master Mix (Applied Biosystems) in an ABI PRISM 7000 sequence detector. The forward and reverse primers were designed using the Oligonucleotide express software computer package. The Cts provided by the software of the equipment were used to analyze the expression using the Excel 5.0 program, calculating the 2^{^(40-Ct)} values and standardizing them with respect to the Actina8 reference gene. The sequences of the primers used are:

| Gene | Sequence |
|---|---|
| forward OCP3 | SEC.ID.NO.13 |
| reverse OCP3 | SEC.ID.NO.14 |
| forward ABI1 | SEC.ID.NO.15 |
| reverse ABI1 | SEC.ID.NO.16 |
| forward ACT8 | SEC.ID.NO.17 |
| reverse ACT8 | SEC.ID.NO.18 |

### Assays of drought resistance.

Twenty plants per genotype were grown in short day conditions for 6 weeks, watering them twice a week with nutritive solution and placing them randomly on different trays. After this time, they were not watered and after 12 days, representative photographs of each genotype were taken. The watering was then re-established and after 48 hours representative photographs were taken again.

### Assays of infection with B.cinerea.

For the inoculations, 5-6 week-old plants grown in short day conditions were used. Five-six leaves/plant were inoculated with 5 µL of a suspension of spores at a final concentration of 10⁶ spores/mL. The inoculated plants were maintained in an atmosphere of high environmental moisture and the diameter of the injury caused by the fungus was measured 72 hours after the inoculation. Twenty plants per genotype were used and the experiments were conducted at least three times.

### Yeast two-hybrid screening

To search for OCP3 interactors, a yeast two-hybrid screening was performed, using a gene library of cDNA of *A.thaliana* in vector pACTII (Clontech) transformed in the PJ69-4α yeast strain, thus expressing the proteins as fusions to the GAL4 (GAL4AD)-activating domain. The cDNA of *OCP3* expressed in the PJ69-4A yeast as a fusion to the GAL4 (GAL4BD)-activating domain in vector pAS2-1 (Clontech) was used as a primer, using the method described by Soellick and Uhrig to determine the interactions. The selection was performed by means of assays of growth of the yeasts in histidine-free medium and with different concentrations of 3-amino-triazole (3AT). To reconfirm the positive clones, the plasmids were extracted from the yeasts, *E.coli* was transformed, they were sequenced and subsequently the yeast was retransformed and the interaction was checked again.

### Measurement of the stomatal opening

To determine the openings of the stomata, completely expanded rosette leaves of 6 week-old plants grown in short day conditions were used. Firstly, the leaves were incubated for 2 hours in an opening-inducing buffer (10 mM Mes, adjusting the pH to 6.15 with KOH, 30 mM KC1). The leaves were incubated with this same buffer supplemented with 100 µM ABA (Sigma) to induce the closure of the stomata. Ten photographs were taken of different peelings of leaves for each genotype, time and treatment with the aid of a digital Nikon DXm1200F camera coupled to a Nikon Eclipse E600 microscope and using the Act-1 software (Nikon). The measurements were made according to the considerations described in *Ichida et al*., using the free ImageJ 1.36b software (Broken Symmetry software). The experiments were conducted 5 times with similar results.

### LITERATURE

1. Abe H, Urao T, Ito T, Seki M, Shinozaki K, Yamaguchi-Shinozaki K. (2003) Arabidopsis AtMYC2 (bHLH) and AtMYB2 (MYB) function as transcriptional activators in abscisic acid signaling. Plant Cell 15: 63-78
2. Allen, G.J., Kuchitsu, K., Chu, S.P., Murata, Y., Schroeder, J.I. (1999) Arabidopsis abi1-1 and abi2-1 phosphatase mutations reduce abscisic acid-induced cytoplasmic calcium rises in guard cells . Plant Cell, 11, 1785 1798.
3. Baker, S.S., Wilhelm, K.S. and Thomashow, M.F. (1994) The 5'-region of Arabidopsis thaliana cor15a has cis-acting elements that confer cold-, drought-, and ABA-regulated gene expression. Plant Mol. Biol. 24, 701-713.
4. Choi, H., Hong, J., Ha, J., Kang, J. and Kim, S.Y. (2000) ABFs, a family of ABA-responsive element binding factors. J. Biol. Chem. 275: 1723-1730.
5. Coego A, Ramirez V, Ellul P, Mayda E, Vera P. (2005a) The H2O2-regulated Ep5C gene encodes a peroxidase required for bacterial speck susceptibility in tomato. Plant J. 42(2):283-93.
6. Coego A, Ramirez V, Gil MJ, Flors V, Mauch-Mani B, Vera P. (2005b) An Arabidopsis homeodomain transcription factor, OVEREXPRESSOR OF CATIONIC PEROXIDASE 3, mediates resistance to infection by necrotrophic pathogens. Plant Cell 17(7):2123-37.
7. Davies, W.J. and Jones, H.G. (1991). Abscisic Acid Physiology and Biochemistry. Oxford: BIOS Scientific Publishers.
8. Finkelstein, R.R. and Somerville, C.R. (1990) Three classes of abscisic acid (ABA) -insensitive mutations of Arabidopsis define genes that control overlapping subsets of ABA responses . Plant Physiol. 94, 1172 1179.
9. Gilmour SJ, Sebolt AM, Salazar MP, Everard JD, Thomashow MF (2000) Overexpression of the Arabidopsis CBF3 transcriptional activator mimics multiple biochemical changes associated with cold acclimation. Plant Physiol 124: 1854-1865.
10. Hetherington AM. (2001) Guard cell signalling. Cell 107, 711-714.
11. Holmberg,N. and Bülow, L. (1998). Improving stress tolerance by gene transfer. Trends in plant science 3 (2).
12. Ichida AM, Pei ZM, Baizabal-Aguirre VM, Turner KJ, Schroeder JI. (1997) Expression of a Cs+-resistant guard cell K+ channel confers Cs+-resistant, light-induced stomatal opening in transgenic Arabidopsis. The Plant Cell 9, 1843-1857.
13. Jefferson R. A., Kavanagh T. A., Bevan M. W. (1987) GUS fusions, beta- glucuronidase as a sensitive and versatile gene fusion marker in higher plants. EMBO J. 6, 3901-3907.
14. Kaffman A, Herskowitz I, Tjian R, O'Shea EK. (1994) Phosphorylation of the transcription factor PHO4 by a cyclin-CDK complex, PHO80-PHO85. Science.25;263(5150):1153-6.
15. Kang, J.Y., Choi, H.I., Im, M.Y., and Kim, S.Y. (2002). Arabidopsis basic leucine zipper proteins that mediate stress-responsive abscisic acid signaling. Plant Cell 14: 343-357
16. Kasuga, M., Liu, Q., Miura, S., Yamaguchi-Shinozaki, K., and Shinozaki, K. (1999) Improving plant drought, salt, and freezing tolerance by gene transfer of a single stress-inducible transcription factor. Nat. Biotechnol. 17:287-291
17. Koornneef, M., Léon-Kloosterziel, K.M., Schwartz, S.H., Zeevaart, J.A.D. (1998) The genetic and molecular dissection of abscisic acid biosynthesis and signal transduction in Arabidopsis . Plant Physiol. Biochem. 36, 83 89
18. Léon-Kloosterziel KM, Gil MA, Ruijs GJ, Jacobsen SE, Olszewski NE, Schwartz SH, Zeevaart JAD, Koornneef M. (1996) Isolation and characterization of abscisic acid-deficient Arabidopsis mutants at two new loci. Plant J. 10: 655-661
19. Leung, J., Merlot, S., Giraudat, J. (1997) The Arabidopsis ABSCISIC ACID-INSENSITIVE 2 (ABI2) and ABI1 genes encode homologous protein phosphatases 2C involved in abscisic acid signal transduction . Plant Cell, 9, 759 771.
20. Liu J, Ishitani M, Halfter U, Kin CS, Zhu J-K. (2000) The Arabidopsis thaliana SOS2 gene encodes a protein kinase that is requires for salt tolerance. Proc Natl Acad Sci USA 97: 3730-3740
21. McAinsh M.R., Brownlee C., Hetherington A.M. (1990) Abscisic acid-induced elevation of guard cell cytosolic Ca2+ precedes stomatal closure. Nature. 343:186-188.
22. Merlot S, Gosti F, Guerrier D, Vavasseur A, Giraudat J. (2001) The ABI1 and ABI2 protein phosphatases 2C act in a negative feedback regulatory loop of the abscisic acid signalling pathway. Plant J. 25(3):295-303.
23. Mishra G, Zhang W, Deng F, Zhao J, Wang X. (2006) A bifurcating pathway directs abscisic acid effects on stomatal closure and opening in Arabidopsis. Science. 2006 14;312(5771):264-6.
24. Nordin K, Vahala T, Palva ET. (1993) Differential expression of two related, low-temperature-induced genes in Arabidopsis thaliana (L.) Heynh. Plant Mol Biol. 21(4):641-53.
25. Nowak SJ and Corces VG (2000) Phosphorylation of histone H3 correlates with transcriptionally active loci. Genes Dev, 14, 3003-3013.
26. Pei, Z., Murata, Y., Benning, G. Thomine, S., Kluesener, B., Allen, G.J., Grill, E. and Schroeder, J.I. (2000) Calcium channels activated by hydrogen peroxide mediate abscisic acid signaling in guard cells. Nature 406: 731-734.
27. Pines, J. (1999) Four-dimensional control of the cell cycle. Nat. Cell Biol. 1:E73-E79.
28. Sakuma, Y., Maruyama, K., Osakabe, Y., Qin, F., Seki, M., Shinozaki, K. and Yamaguchi-Shinozaki, K. (2006) Functional analysis of an Arabidopsis transcription factor, DREB2A, involved in drought-responsive gene expression. Plant Cell 18, 1292-1309.
29. Sanders, D., Brownlee, C. and Harper, J.F. (1999) Communicating with calcium. Plant Cell 11: 691-706.
30. Schroeder JI, Allen GJ, Hugouvieux V, Kwak JM, Waner D. (2001) Guard cell signal transduction. Annual Review of Plant Physiology and Plant Molecular Biology 52, 627-658.
31. Seki M, Kamei A, Yamaguchi-Shinozaki K, Shinozaki K. (2003) Molecular responses to drought, salinity and frost: common and different paths for plant protection. Curr Opin Biotechnol 14: 194-199.
32. Shinozaki, K. and Yamaguchi-Shinozaki, K. (2000) Molecular responses to dehydration and low temperature: differences and cross-talk between two stress signaling pathways. Curr. Opin. Plant Biol. 3: 217-223.
33. Soellick, T.R. and Uhrig, J.F. (2001) Development of an optimized interaction-mating protocol for large-scale yeast two-hybrid analyses. Genome Biol. 2, 00521-00527
34. Umezawa T, Okamoto M, Kushiro T, Nambara E, Oono Y, Seki M, Kobayashi M, Koshiba T, Kamiya Y, Shinozaki K. (2006) CYP707A3, a major ABA 8'-hydroxylase involved in dehydration and rehydration response in Arabidopsis thaliana. Plant J. 46(2):171-82.
35. Uno, Y., Furihata, T., Abe, H., Yoshida, R., Shinozaki, K. and Yamaguchi-Shinozaki, K. (2000) Arabidopsis basic leucine zipper transcription factors involved in an abscisic acid-dependent signal transduction pathway under drought and high-salinity conditions. Proc. Natl. Acad. Sci. USA 97: 11632-11637.
36. Weigel D. (1995) The APETALA2 domain is related to a novel type of DNA binding domain. Plant Cell 7: 388-389
37. Whitmarsh, A. J., and R. J. Davis. 2000. Regulation of transcription factor function by phosphorylation. Cell. Mol. Life Sci. 57:1172-1183.
38. Xiong, L., Ishitani, M., Lee, H. and Zhu, J.K. (2001) The Arabidopsis LOS5/ABA3 locus encodes a molybdenum cofactor sulfurase and modulates cold stress- and osmotic stress-responsive gene expression. Plant Cell 13: 2063-2083.
39. Yamaguchi-Shinozaki, K. and Shinozaki, K. (1994) A novel cis-acting element in an Arabidopsis gene is involved in responsiveness to drought, low-temperature, or high-salinity stress. Plant Cell, 6, 251-264.
40. Zeevaart, J.A.D. and Creelman, R.A. (1988) Metabolism and physiology of abscisic acid. Annu. Rev. Plant Physiol. Plant Mol. Biol. 39, 439 473.

## Claims

1. Use of the *ocp3* loss of function mutation of the OCP3 gene in plant species of agronomic and industrial interest as a regulator of the response of plants to drought, to confer drought resistance or increased drought tolerance.

2. Use of the *ocp3* loss of function mutation of the OCP3 gene according to claim 1, to confer drought resistance in plants by means of the inhibition of its expression by means of gene silencing.

3. Use of the *ocp3* loss of function mutation of the OCP3 gene according to claim 1, to confer drought resistance in plants by means of the loss of its functionality by means of chemical mutagenesis.

4. Genetically modified plants in which OCP3 gene has been manipulated such that the expression of such gene is inhibited or it's function lossed, conferring to said plants drought resistance or greater tolerance to drought.


SEQUENCE LISTING

<110> CALANTIA BIOTECH, S.L

<120> Use of the ocp3 mutation to regulate drought resistance in plants

<130> AX090051EP

<150> P200700128

<151> 2006-10-31

<160> 20

<170> PatentIn version 3.3

<210> 1

<211> 19

<212> DNA

<213> Artificial

<220>

<223> PoRT1 primer

<400> 1

ttgagaaagt agagaatgg 19

<210> 2

<211> 21

<212> DNA

<213> Artificial

<220>

<223>  PoRT2 primer

<400>  2
gtatcccagt ttcacatgta g                                                21

<210>  3
<211>  22
<212>  DNA
<213>  Artificial

<220>
<223> tomOCP3EcoRI primer

<400>  3
gtagagaatt ccgtcttctt cc                                               22

<210>  4
<211>  21
<212>  DNA
<213>  Artificial

<220>
<223> tomOCP3XhoI primer

<400>  4

ttcaagcttc tcgaggtcct c                                                21

<210>  5
<211>  21
<212>  DNA
<213>  Artificial

<220>
<223>  rd29A direct primer

<400>  5
tgcaccaggc gtaacaggta a                                                21

<210>  6
<211>  22
<212>  DNA
<213>  Artificial

<220>
<223>  rd29A reverse primer

<400>  6
ttgtccgatg taaacgtcgt cc                                               22

<210>  7

<211>  22

<212>  DNA

<213>  Artificial

<220>

<223>  rd29B direct primer

<400>  7

gcgcaccagt gtatgaatcc tc                                                22

<210>  8

<211>  22

<212>  DNA

<213>  Artificial

<220>

<223>  rd29B reverse primer

<400>  8

tgtggtcaga agacacgaca gg                                                22

<210>  9

<211>  22

<212>  DNA

<213>  Artificial

<220>

<223>  rd22 direct primer

<400>  9

aggtggctaa gaagaacgca cc                                                22

<210>  10

<211>  21

<212>  DNA

<213>  Artificial

<220>

<223>  rd22 reverse primer

<400>  10

tggcagtaga acaccgcgaa t                                                 21

<210>  11

<211>  20

<212>  DNA

<213>  Artificial

<220>

<223>  ELF1alfa direct primer

<400>  11
gcacagtcat tgatgcccca                                                    20

<210>  12
<211>  21
<212>  DNA
<213>  Artificial

<220>
<223>  ELF1alfa reverse primer

<400>  12
cctcaagaag agttggtccc t                                                  21

<210>  13
<211>  24
<212>  DNA
<213>  Artificial

<220>
<223>  OCP3 direct primer

<400>  13

aagctgggcg tcgtaaaact agta 24

<210> 14

<211> 24

<212> DNA

<213> Artificial

<220>

<223> OCP3 reverse primer

<400> 14

tggcggtttt tcatctggta gtgt 24

<210> 15

<211> 22

<212> DNA

<213> Artificial

<220>

<223> ABI1 direct primer

<400> 15

gtcgagatcc attggcgata ga 22

<210>  16

<211>  21

<212>  DNA

<213>  Artificial

<220>

<223>  ABI1 reverse primer

<400>  16

tgccatctca cacgcttctt c                                                21

<210>  17

<211>  23

<212>  DNA

<213>  Artificial

<220>

<223>  ACT8 direct primer

<400>  17

agtggtcgta caaccggtat tgt                                              23

<210>  18

<211>  24

<212>  DNA

<213>  Artificial

<220>

<223>  ACT8 reverse primer

<400>  18

gaggatagca tgtggaagtg agaa                                              24

<210>  19

<211>  21

<212>  DNA

<213>  Artificial

<220>

<223>  OCP3-1 primer

<400>  19

gaattcatga taaaagccat g                                                 21

<210>  20

<211>  24

<212>  DNA

<213>  Artificial

<220>

<223>  OCP3-2 primer

<400>  20
gttaacatta gatcacccgg gagc                                              24